# EUROPEAN PATENT SPECIFICATION

(11) **EP 4 017 578 B1**
(45) Date of publication and mention of the grant of the patent: **10.05.2023**
(21) Application number: 20767670.1
(22) Date of filing: 21.08.2020
(51) Int. Cl.: A61N 1/36, A61B 5/06, A61N 1/04, A61N 1/05

(54) **ELECTRODE IMPEDANCE BASED DETECTION OF TRANSLOCATION OF AN ELECTRODE LEAD WITHIN A COCHLEA**
ELEKTRODENIMPEDANZBASIERTE DETEKTION DER TRANSLOKATION EINER ELEKTRODENLEITUNG IN EINER COCHLEA
DÉTECTION BASÉE SUR L'IMPÉDANCE D'ÉLECTRODE DE LA TRANSLOCATION D'UN FIL D'ÉLECTRODE À L'INTÉRIEUR D'UNE COCHLÉE

(30) Priority: 23.08.2019 US 201962891053 P
(43) Date of publication of application: 29.06.2022
(73) Proprietor: Advanced Bionics AG, 8712 Staefa (CH); Academisch Ziekenhuis Leiden, 2333 ZA Leiden (NL)
(72) Inventor: FRIJNS, Johannes H.M., 2300 RC Leiden (NL); BRIAIRE, Jeroen J., 2333 ZA Leiden (NL); BOYLE, Patrick J., Kent BR3 1LZ (GB); HAMACHER, Volkmar, 30657 Hannover (DE); LITVAK, Leonid M., Los Angeles, California 90038 (US)
(74) Representative: Schwan Schorer & Partner mbB
(86) International application number: PCT/US2020/047461
(87) International publication number: WO 2021/041236

(56) References cited:
- EP-A1- 3 046 618
- WO-A1-2017/160948
- US-A1- 2019 240 489

## Description

### BACKGROUND INFORMATION

Correct insertion and placement of an electrode lead within a cochlea for use with a cochlear implant is of great importance for effective electrical stimulation and effective use of the cochlear implant. For example, it is important for the electrode lead to stay within the scala tympani of the cochlea instead of translocating to the scala vestibuli, to be oriented correctly, and to minimize trauma to intracochlear structures so as to preserve any residual hearing that a cochlear implant recipient may have.

Unfortunately, current methods for detecting electrode lead translocation typically involve imaging technology (e.g., x-ray technology, fluoroscopic technology, computerized tomography (CT) scanning technology, etc.) that is expensive, inconvenient, and impractical or impossible to employ in real time during lead insertion procedures. A system according to the preamble of claim 1 known from US2019/240489.

### BRIEF DESCRIPTION OF THE DRAWINGS

The accompanying drawings illustrate various embodiments and are a part of the specification. The illustrated embodiments are merely examples and do not limit the scope of the disclosure. Throughout the drawings, identical or similar reference numbers designate identical or similar elements.
FIG. 1 illustrates an exemplary cochlear implant system.
FIG. 2 shows an exemplary configuration of the cochlear implant system of FIG. 1.
FIG. 3 shows another exemplary configuration of the cochlear implant system of FIG. 1.
FIG. 4 shows another exemplary configuration of the cochlear implant system of FIG. 1.
FIG. 5 shows exemplary aspects of an electrode lead and of recipient anatomy as an exemplary insertion procedure is performed.
FIG. 6 shows an exemplary insertion management system.
FIG. 7 shows a distal portion of an electrode lead.
FIG. 8 shows various types of impedances that may be measured by the system of FIG. 6.
FIG. 9 shows an exemplary implementation of the system of FIG. 6.
FIG. 10 shows an exemplary configuration in which a machine learning model is trained.
FIG. 11 illustrates an exemplary method.
FIG. 12 illustrates an exemplary computing device.

### DETAILED DESCRIPTION

The invention relates to a system as defined in claim 1. Any methods disclosed hereinafter do not form part of the scope of the invention and are mentioned for illustrative purposes only. Systems for detecting translocation (also referred to herein as "scalar translocation" and "electrode lead translocation") or potential translocation of an electrode lead during a lead insertion procedure are described herein. Detecting that scalar translocation is about to occur or that it has occurred may assist in detecting potential and/or actual cochlear trauma and/or identifying the position and/or insertion path of an electrode lead with respect to a cochlea into which the electrode lead is inserted during a lead insertion procedure. As used herein, translocation of an electrode lead refers to a movement by the electrode lead (e.g., a distal portion of the electrode lead, in particular) from one scala (e.g., the scala tympani) of the cochlea of a recipient to another scala (e.g., the scala vestibuli) of the cochlea of the recipient.

For example, during a lead insertion procedure in which an electrode lead is inserted into a cochlea, the electrode lead may travel into the scala tympani of the cochlea but, instead of continuing to travel through the scala tympani, may inadvertently puncture the basilar membrane and/or other anatomy separating the scala tympani from the scala vestibuli to enter the scala vestibuli. Because such a scalar translocation may damage the basilar membrane and hair cells disposed on the basilar membrane, the translocation of the electrode lead may cause trauma to the cochlea and potentially adversely affect the recipient's residual hearing, provoke more reaction to the foreign body (e.g., the electrode lead), produce more fibrous sheath, increase impedances, and/or lead to the loss of cochlear tissue that could have an adverse effect on use of the cochlear implant. As such, systems and methods described herein are configured to detect scalar translocation in real time during the lead insertion procedure (e.g., so that the scalar translocation may be corrected if possible) and/or after the lead insertion procedure (e.g., so that the scalar translocation may be associated with data being studied to help reduce trauma and improve outcomes in subsequent lead insertion procedures, or for other suitable purposes as described herein).

Systems described herein for detecting scalar translocation of an electrode lead within a cochlea of a cochlear implant recipient may provide various benefits to cochlear implant recipients, as well as to surgeons and others involved with insertion procedures. For example, by providing real time information about whether a scalar translocation of the electrode lead or other trauma is occurring during an insertion procedure, disclosed systems may provide a surgeon or other user performing the insertion procedure with information and perspective into the intricate insertion procedure, thereby allowing for a translocated electrode lead to be corrected (e.g., withdrawn and reinserted without scalar translocation) or for trauma to otherwise be mitigated to facilitate a successful outcome of the lead insertion procedure.

Along with providing perspective into the insertion procedure (e.g., informing surgeons and surgical team members which scala(s) an electrode lead being inserted is currently located in), disclosed systems may further provide data representative of whether a scalar translocation of the electrode lead is about to occur has occurred, which scala the electrode lead is currently located in, and so forth, to computer systems used to facilitate the insertion procedure. This may allow the computer systems to provide feedback or warnings (e.g., by way of user interfaces, lights, sounds, etc.) that may help the surgeon and other people involved in performing the insertion procedure to proceed with appropriate care at various stages of the procedure. Moreover, in situations where variables such as procedure time, electrode lead location (e.g., current insertion depth), and the like are being tracked along with the cochlear trauma, computer systems may log trauma events to correlate with these other variables to be used in subsequent procedures for other cochlear implant recipients (e.g., to warn surgeons to take particular care at particular times or insertion depths, to perform particular actions when an electrode lead is coming up on a depth where a scalar translocation of the electrode lead has previously occurred, etc.).

Even after an insertion procedure is complete, disclosed systems for detecting scalar translocation of electrode leads may be useful for providing insight into a final resting location at which the electrode lead has been inserted.

Additionally, regardless of whether disclosed system for detecting scalar translocation of an electrode lead are performed in real time during an insertion procedure or after the fact when the electrode lead is stationary, the detecting of scalar translocation without use of expensive, inconvenient, or risky imaging technology (e.g., x-ray technology, fluoroscopic technology, CT scanning technology, etc.) may be beneficial. For example, by detecting the scalar translocation of the electrode lead while avoiding these other technologies, recipients may be less exposed to various risks, inconveniences, costs, and/or other undesirable aspects associated with such technology.

Various embodiments will now be described in more detail with reference to the figures. The disclosed systems may provide one or more of the benefits mentioned above and/or various additional and/or alternative benefits that will be made apparent herein.

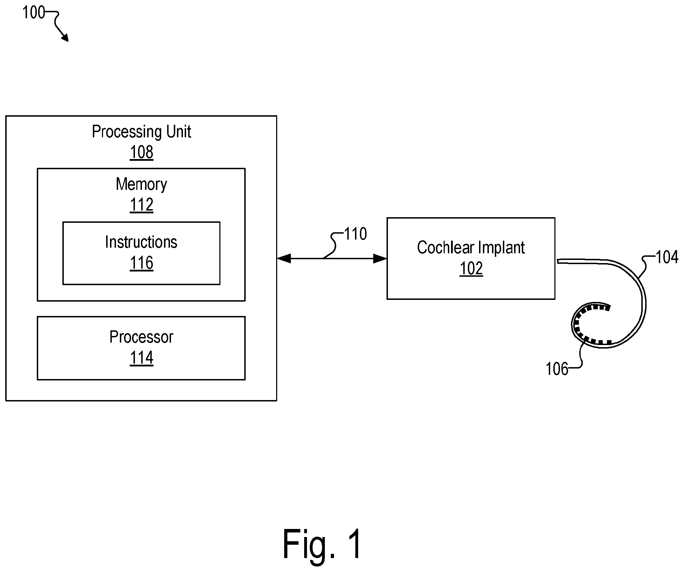

FIG. 1 illustrates an exemplary cochlear implant system 100 configured to be used by a recipient. As shown, cochlear implant system 100 includes a cochlear implant 102, an electrode lead 104 physically coupled to cochlear implant 102 and having an array of electrodes 106, and a processing unit 108 configured to be communicatively coupled to cochlear implant 102 by way of a communication link 110.

The cochlear implant system 100 shown in FIG. 1 is unilateral (i.e., associated with only one ear of the recipient). Alternatively, a bilateral configuration of cochlear implant system 100 may include separate cochlear implants and electrode leads for each ear of the recipient. In the bilateral configuration, processing unit 108 may be implemented by a single processing unit configured to interface with both cochlear implants or by two separate processing units each configured to interface with a different one of the cochlear implants.

Cochlear implant 102 may be implemented by any suitable type of implantable stimulator. For example, cochlear implant 102 may be implemented by an implantable cochlear stimulator. Additionally or alternatively, cochlear implant 102 may be implemented by a brainstem implant and/or any other type of device that may be implanted within the recipient and configured to apply electrical stimulation to one or more stimulation sites located along an auditory pathway of the recipient.

In some examples, cochlear implant 102 may be configured to generate electrical stimulation representative of an audio signal (also referred to herein as audio content) processed by processing unit 108 in accordance with one or more stimulation parameters transmitted to cochlear implant 102 by processing unit 108. Cochlear implant 102 may be further configured to apply the electrical stimulation to one or more stimulation sites (e.g., one or more intracochlear locations) within the recipient by way of one or more electrodes 106 on electrode lead 104. In some examples, cochlear implant 102 may include a plurality of independent current sources each associated with a channel defined by one or more of electrodes 106. In this manner, different stimulation current levels may be applied to multiple stimulation sites simultaneously by way of multiple electrodes 106.

Cochlear implant 102 may additionally or alternatively be configured to generate, store, and/or transmit data. For example, cochlear implant may use one or more electrodes 106 to record one or more signals (e.g., one or more voltages, impedances, evoked responses within the recipient, and/or other measurements) and transmit, by way of communication link 110, data representative of the one or more signals to processing unit 108. In some examples, this data is referred to as back telemetry data.

Electrode lead 104 may be implemented in any suitable manner. For example, a distal portion of electrode lead 104 may be pre-curved such that electrode lead 104 conforms with the helical shape of the cochlea after being implanted. Electrode lead 104 may alternatively be naturally straight or of any other suitable configuration.

In some examples, electrode lead 104 includes a plurality of wires (e.g., within an outer sheath) that conductively couple electrodes 106 to one or more current sources within cochlear implant 102. For example, if there are n electrodes 106 on electrode lead 104 and n current sources within cochlear implant 102, there may be n separate wires within electrode lead 104 that are configured to conductively connect each electrode 106 to a different one of the n current sources. Exemplary values for n are 8, 12, 16, or any other suitable number.

Electrodes 106 are located on at least a distal portion of electrode lead 104. In this configuration, after the distal portion of electrode lead 104 is inserted into the cochlea, electrical stimulation may be applied by way of one or more of electrodes 106 to one or more intracochlear locations. One or more other electrodes (e.g., including a ground electrode, not explicitly shown) may also be disposed on other parts of electrode lead 104 (e.g., on a proximal portion of electrode lead 104) to, for example, provide a current return path for stimulation current applied by electrodes 106 and to remain external to the cochlea after the distal portion of electrode lead 104 is inserted into the cochlea. Additionally or alternatively, a housing of cochlear implant 102 may serve as a ground electrode for stimulation current applied by electrodes 106.

Processing unit 108 may be configured to interface with (e.g., control and/or receive data from) cochlear implant 102. For example, processing unit 108 may transmit commands (e.g., stimulation parameters and/or other types of operating parameters in the form of data words included in a forward telemetry sequence) to cochlear implant 102 by way of communication link 110. Processing unit 108 may additionally or alternatively provide operating power to cochlear implant 102 by transmitting one or more power signals to cochlear implant 102 by way of communication link 110. Processing unit 108 may additionally or alternatively receive data from cochlear implant 102 by way of communication link 110. Communication link 110 may be implemented by any suitable number of wired and/or wireless bidirectional and/or unidirectional links.

As shown, processing unit 108 includes a memory 112 and a processor 114 configured to be selectively and communicatively coupled to one another. In some examples, memory 112 and processor 114 may be distributed between multiple devices and/or multiple locations as may serve a particular implementation.

Memory 112 may be implemented by any suitable non-transitory computer-readable medium and/or non-transitory processor-readable medium, such as any combination of non-volatile storage media and/or volatile storage media. Exemplary non-volatile storage media include, but are not limited to, read-only memory, flash memory, a solid-state drive, a magnetic storage device (e.g., a hard drive), ferroelectric random-access memory ("RAM"), and an optical disc. Exemplary volatile storage media include, but are not limited to, RAM (e.g., dynamic RAM).

Memory 112 may maintain (e.g., store) executable data used by processor 114 to perform one or more of the operations described herein as being performed by processing unit 108. For example, memory 112 may store instructions 116 that may be executed by processor 114 to perform any of the audio content processing and cochlear implant control operations described herein. Instructions 116 may be implemented by any suitable application, program (e.g., sound processing program), software, code, and/or other executable data instance. Memory 112 may also maintain any data received, generated, managed, used, and/or transmitted by processor 114.

Processor 114 may be configured to perform (e.g., execute instructions 116 stored in memory 112 to perform) various operations with respect to cochlear implant 102.

To illustrate, processor 114 may be configured to control an operation of cochlear implant 102. For example, processor 114 may receive an audio signal (e.g., by way of a microphone communicatively coupled to processing unit 108, a wireless interface (e.g., a Bluetooth interface), and/or a wired interface (e.g., an auxiliary input port)). Processor 114 may process the audio signal in accordance with a sound processing strategy (e.g., a sound processing program stored in memory 112) to generate appropriate stimulation parameters. Processor 114 may then transmit the stimulation parameters to cochlear implant 102 to direct cochlear implant 102 to apply electrical stimulation representative of the audio signal to the recipient.

Processor 114 may be additionally or alternatively configured to receive and process data generated by cochlear implant 102. For example, processor 114 may receive data representative of a signal recorded by cochlear implant 102 using one or more electrodes 106 and, based on the data, adjust one or more operating parameters of processing unit 108. Additionally or alternatively, processor 114 may use the data to perform one or more diagnostic operations with respect to cochlear implant 102 and/or the recipient.

Other operations may be performed by processor 114 as may serve a particular implementation. In the description provided herein, any references to operations performed by processing unit 108 and/or any implementation thereof may be understood to be performed by processor 114 based on instructions 116 stored in memory 112.

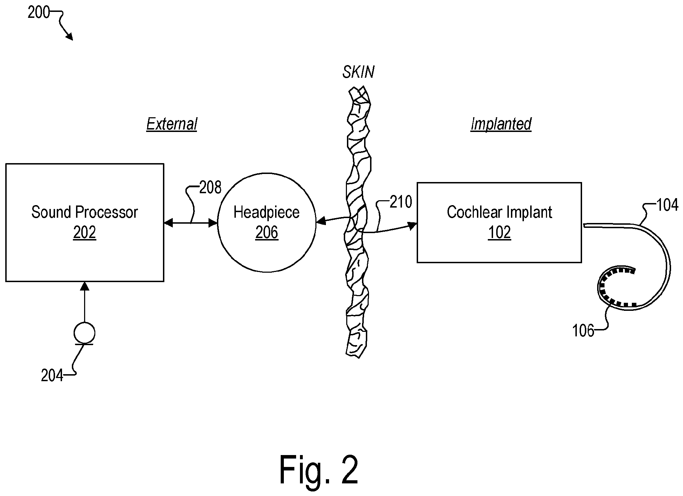

Processing unit 108 may be implemented by one or more devices configured to interface with cochlear implant 102. To illustrate, FIG. 2 shows an exemplary configuration 200 of cochlear implant system 100 in which processing unit 108 is implemented by a sound processor 202 configured to be located external to the recipient. In configuration 200, sound processor 202 is communicatively coupled to a microphone 204 and to a headpiece 206 that are both configured to be located external to the recipient.

Sound processor 202 may be implemented by any suitable device that may be worn or carried by the recipient. For example, sound processor 202 may be implemented by a behind-the-ear ("BTE") unit configured to be worn behind and/or on top of an ear of the recipient. Additionally or alternatively, sound processor 202 may be implemented by an off-the-ear unit (also referred to as a body worn device) configured to be worn or carried by the recipient away from the ear. Additionally or alternatively, at least a portion of sound processor 202 is implemented by circuitry within headpiece 206.

Microphone 204 is configured to detect one or more audio signals (e.g., that include speech and/or any other type of sound) in an environment of the recipient. Microphone 204 may be implemented in any suitable manner. For example, microphone 204 may be implemented by a microphone that is configured to be placed within the concha of the ear near the entrance to the ear canal, such as a T-MIC^{™} microphone from Advanced Bionics. Such a microphone may be held within the concha of the ear near the entrance of the ear canal during normal operation by a boom or stalk that is attached to an ear hook configured to be selectively attached to sound processor 202. Additionally or alternatively, microphone 204 may be implemented by one or more microphones in or on headpiece 206, one or more microphones in or on a housing of sound processor 202, one or more beam-forming microphones, and/or any other suitable microphone as may serve a particular implementation.

Headpiece 206 may be selectively and communicatively coupled to sound processor 202 by way of a communication link 208 (e.g., a cable or any other suitable wired or wireless communication link), which may be implemented in any suitable manner. Headpiece 206 may include an external antenna (e.g., a coil and/or one or more wireless communication components) configured to facilitate selective wireless coupling of sound processor 202 to cochlear implant 102. Headpiece 206 may additionally or alternatively be used to selectively and wirelessly couple any other external device to cochlear implant 102. To this end, headpiece 206 may be configured to be affixed to the recipient's head and positioned such that the external antenna housed within headpiece 206 is communicatively coupled to a corresponding implantable antenna (which may also be implemented by a coil and/or one or more wireless communication components) included within or otherwise connected to cochlear implant 102. In this manner, stimulation parameters and/or power signals may be wirelessly and transcutaneously transmitted between sound processor 202 and cochlear implant 102 by way of a wireless communication link 210.

In configuration 200, sound processor 202 may receive an audio signal detected by microphone 204 by receiving a signal (e.g., an electrical signal) representative of the audio signal from microphone 204. Sound processor 202 may additionally or alternatively receive the audio signal by way of any other suitable interface as described herein. Sound processor 202 may process the audio signal in any of the ways described herein and transmit, by way of headpiece 206, stimulation parameters to cochlear implant 102 to direct cochlear implant 102 to apply electrical stimulation representative of the audio signal to the recipient.

In an alternative configuration, sound processor 202 may be implanted within the recipient instead of being located external to the recipient. In this alternative configuration, which may be referred to as a fully implantable configuration of cochlear implant system 100, sound processor 202 and cochlear implant 102 may be combined into a single device or implemented as separate devices configured to communicate one with another by way of a wired and/or wireless communication link. In a fully implantable implementation of cochlear implant system 100, headpiece 206 may not be included and microphone 204 may be implemented by one or more microphones implanted within the recipient, located within an ear canal of the recipient, and/or external to the recipient.

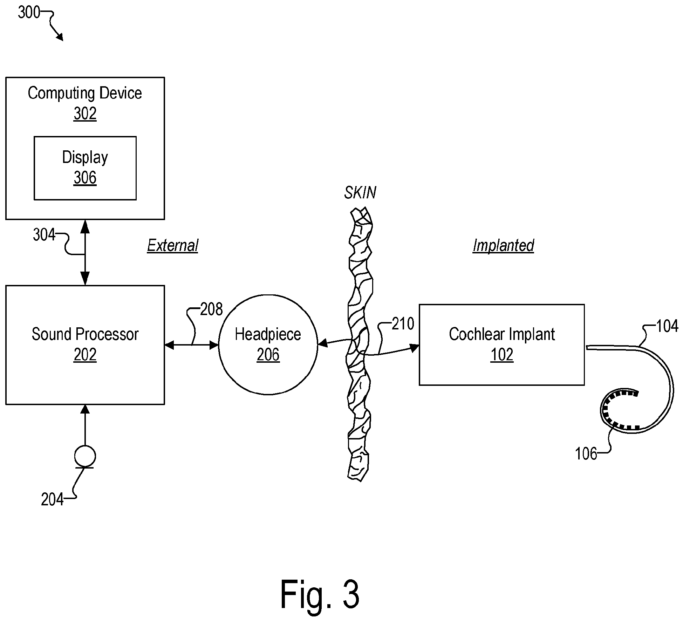

FIG. 3 shows an exemplary configuration 300 of cochlear implant system 100 in which processing unit 108 is implemented by a combination of sound processor 202 and a computing device 302 configured to communicatively couple to sound processor 202 by way of a communication link 304, which may be implemented by any suitable wired or wireless communication link.

Computing device 302 may be implemented by any suitable combination of hardware and software. To illustrate, computing device 302 may be implemented by a mobile device (e.g., a mobile phone, a laptop, a tablet computer, etc.), a desktop computer, and/or any other suitable computing device as may serve a particular implementation. As an example, computing device 302 may be implemented by a mobile device configured to execute an application (e.g., a "mobile app") that may be used by a user (e.g., the recipient, a clinician, and/or any other user) to control one or more settings of sound processor 202 and/or cochlear implant 102 and/or perform one or more operations (e.g., diagnostic operations) with respect to data generated by sound processor 202 and/or cochlear implant 102.

In some examples, computing device 302 may be configured to control an operation of cochlear implant 102 by transmitting one or more commands to cochlear implant 102 by way of sound processor 202. Likewise, computing device 302 may be configured to receive data generated by cochlear implant 102 by way of sound processor 202. Alternatively, computing device 302 may interface with (e.g., control and/or receive data from) cochlear implant 102 directly by way of a wireless communication link between computing device 302 and cochlear implant 102. In some implementations in which computing device 302 interfaces directly with cochlear implant 102, sound processor 202 may or may not be included in cochlear implant system 100.

Computing device 302 is shown as having an integrated display 306. Display 306 may be implemented by a display screen, for example, and may be configured to display content generated by computing device 302. Additionally or alternatively, computing device 302 may be communicatively coupled to an external display device (not shown) configured to display the content generated by computing device 302.

In some examples, computing device 302 represents a fitting device configured to be selectively used (e.g., by a clinician) to fit sound processor 202 and/or cochlear implant 102 to the recipient. In these examples, computing device 302 may be configured to execute a fitting program configured to set one or more operating parameters of sound processor 202 and/or cochlear implant 102 to values that are optimized for the recipient. As such, in these examples, computing device 302 may not be considered to be part of cochlear implant system 100. Instead, computing device 302 may be considered to be separate from cochlear implant system 100 such that computing device 302 may be selectively coupled to cochlear implant system 100 when it is desired to fit sound processor 202 and/or cochlear implant 102 to the recipient.

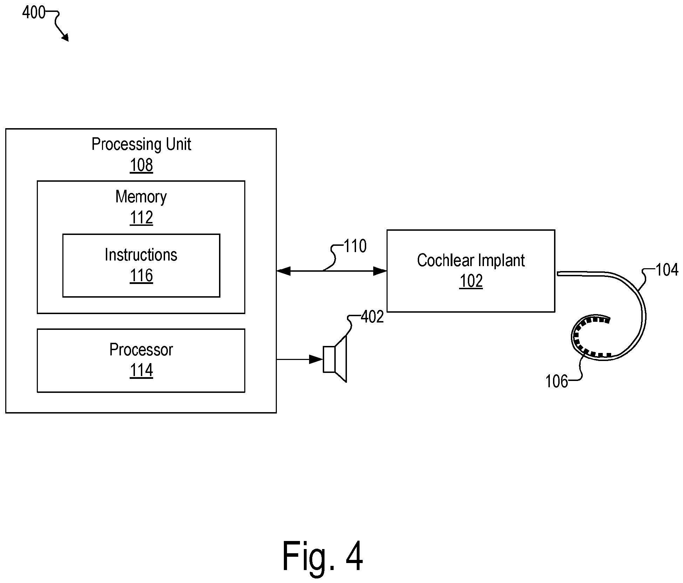

In some implementations, processing unit 108 may also be configured to apply acoustic stimulation to the recipient. For example, FIG. 4 shows an exemplary configuration 400 in which a receiver 402 (also referred to as a loudspeaker) may be coupled to processing unit 108. Receiver 402 may be coupled to processing unit 108 in any suitable manner. For example, receiver 402 may be coupled directly to sound processor 202 or to computing device 302.

In configuration 400, processing unit 108 may deliver acoustic stimulation to the recipient by way of receiver 402. The acoustic stimulation may be representative of an audio signal (e.g., an amplified version of the audio signal) configured to elicit an evoked response within the recipient and/or otherwise configured. In configurations in which processing unit 108 is configured to both deliver acoustic stimulation to the recipient and direct cochlear implant 102 to apply electrical stimulation to the recipient, cochlear implant system 100 may be referred to as a bimodal hearing system and/or any other suitable term.

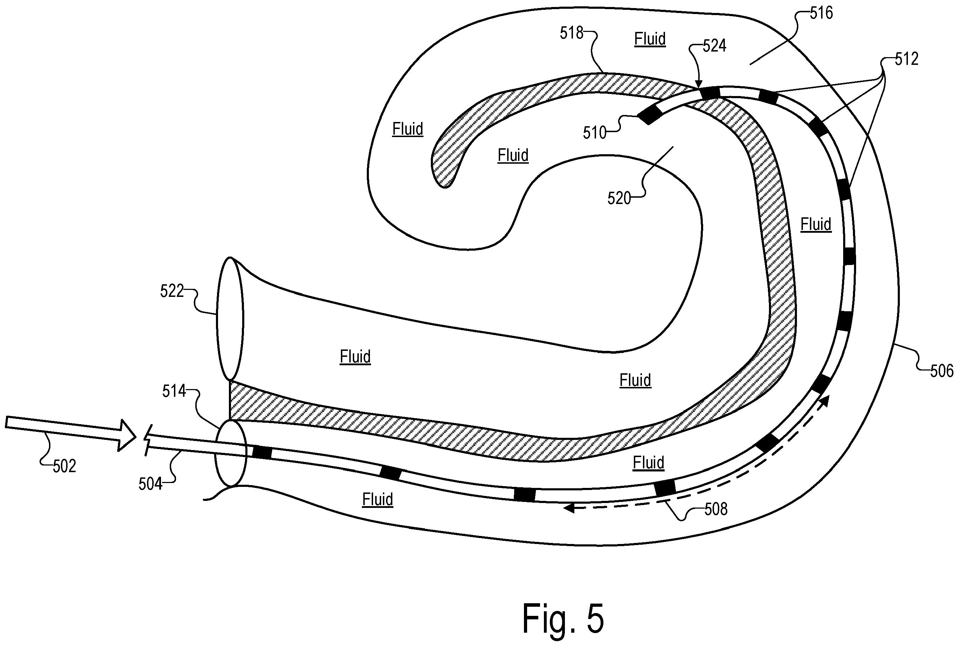

To illustrate the context in which a lead insertion procedure (or simply "insertion procedure") is performed and how a scalar translocation of an electrode lead may occur, FIG. 5 shows exemplary aspects of an electrode lead and of recipient anatomy as an exemplary insertion procedure is performed. Specifically, as shown, an insertion procedure 502 is illustrated in which a distal portion of an electrode lead 504 is inserted into a cochlea 506 of a recipient along an insertion path 508 (which is illustrated in part by a dashed curve but will be understood to including the entire path taken by electrode lead 504 within cochlea 506). It will be understood that, while only a distal portion of electrode lead 504 is illustrated in FIG. 5, a proximal portion of the electrode lead not explicitly shown may be coupled to a cochlear implant (also not shown) that may direct current into electrode lead 504, receive and pass on data detected by electrode lead 504 (e.g., evoked response data or the like), and so forth.

As shown, electrode lead 504 may include various electrodes including a leading electrode 510 (also referred to as a most apical electrode) nearest a distal end of electrode lead 504 and several additional electrodes 512 disposed along the length of electrode lead 504. Unless the context dictates otherwise, it will be understood that electrodes 512, when referred to generally herein, may include all the electrodes disposed on electrode lead 504 including electrode 510 and/or electrodes not explicitly shown in FIG. 5.

As shown, electrode 510 is located at a distal end of lead 504. Thus, electrode 510 may be referred to as a "tip electrode". Alternatively, the distal-most electrode on lead 504 may not be located at the very distal end of lead 504. In these examples, the tip of lead 504 may be made out of silicone or some other insulative material. In cases where electrode 510 is a tip electrode, the tip electrode may be smaller than the other electrodes on lead 504 and may therefore have a higher impedance to start out with. This difference in size and impedance may be corrected or compensated for during the various impedance measurement techniques described herein.

As illustrated in FIG. 5, insertion procedure 502 may involve inserting electrode lead 504 through an entry point 514 (e.g., within a round window or cochleostomy of cochlea 506, or another suitable location) and into a scala tympani 516 of cochlea 506. Scala tympani 516 is a chamber of cochlea 506 that is separated by a basilar membrane 518 (e.g., as well as other membranes and anatomical structures not explicitly shown or labeled in FIG. 5) from a scala vestibuli 520 of cochlea 506 (i.e., a separate chamber of the cochlea). As such, vibrations introduced at an oval window 522 of cochlea 506 may vibrate through fluid included in scala vestibuli 520 toward the apex of cochlea 506 and back toward the base of cochlea 506 through fluid included in scala tympani 516. In other words, sound vibrations traveling on either side of basilar membrane 518 may be moving in opposite directions and, as such, may be out of phase with one another. As the vibrations travel through fluid in scala tympani 516, the vibrations may be detected and encoded by hair cells along basilar membrane 518 (if undamaged hair cells are present in the particular recipient). Additionally or alternatively, electrodes 512 disposed throughout scala tympani 516 may generate electrical stimulation to stand in for the function of damaged hair cells. Regardless, nerves associated with different depths (frequency regions) along cochlea 506 may send signals to the brain to effect a hearing sensation.

FIG. 5 illustrates electrode lead 504 within cochlea 506 at a particular moment during insertion procedure 502. Specifically, at the moment depicted in FIG. 5, electrode lead 504 has translocated from scala tympani 516, through basilar membrane 518, and into scala vestibuli 520 at a translocation site 524. This scalar translocation of electrode lead 504 may have occurred for any of a variety of reasons during insertion procedure 502, but is most likely an undesirable occurrence because, as shown, the distal end of electrode lead 504 (i.e., at leading electrode 510) has physically penetrated basilar membrane 518, thereby potentially causing trauma to basilar membrane 518 and/or any of various other parts of cochlea 506 associated with basilar membrane 518 (e.g., previously functional hair cells along basilar membrane 518, other membranes or nerves associated with basilar membrane 518, etc.).

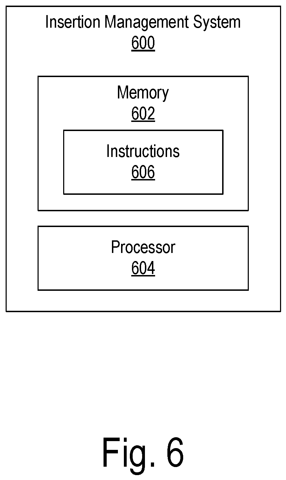

FIG. 6 shows an exemplary insertion management system 600 ("system 600") that may be configured to perform various operations with respect to a lead insertion procedure. In particular, system 600 may be configured to detect that a translocation event in which an electrode lead (e.g., lead 104 or lead 504) translocates from a first scala of the cochlea to a second scala of the cochlea is about to occur or has occurred during a lead insertion procedure. By detecting translocation events, system 600 may be configured to mitigate trauma caused by scalar translocation of an electrode lead and/or facilitate avoidance of similar scalar translocations in future lead insertion procedures.

System 600 may be implemented by one or more computing devices, such as any of the computing devices described herein (e.g., processing unit 108, sound processor 202, and/or computing device 302) and/or any computing device not included in cochlear implant system 100. For example, system 600 may be implemented by one or more computing devices accessible by a user before and/or during a lead insertion procedure and/or one or more servers located remote from an intraoperative space associated with the lead insertion procedure. System 600 may be maintained and/or otherwise associated with a manufacturer of cochlear implant systems, a provider of cochlear implant systems, a surgical center where lead insertion procedures are performed, and/or any other entity as may serve a particular implementation.

As shown, system 600 includes a memory 602 and a processor 604 configured to be selectively and communicatively coupled to one another. In some examples, memory 602 and processor 604 may be distributed between multiple devices and/or multiple locations as may serve a particular implementation.

Memory 602 may be implemented by any suitable non-transitory computer-readable medium and/or non-transitory processor-readable medium, such as any combination of non-volatile storage media and/or volatile storage media as described herein.

Memory 602 may maintain (e.g., store) executable data used by processor 604 to perform one or more of the operations described herein as being performed by system 600. For example, memory 602 may store instructions 606 that may be executed by processor 604 to perform any of the machine learning model operations described herein. Instructions 606 may be implemented by any suitable application, program, software, code, and/or other executable data instance. Memory 602 may also maintain any data received, generated, managed, used, and/or transmitted by processor 604.

Processor 604 may be configured to perform (e.g., execute instructions 606 stored in memory 602 to perform) various operations with respect to detecting a translocation event. In the description provided herein, any references to operations performed by system 600 and/or any implementation thereof may be understood to be performed by processor 604 based on instructions 606 stored in memory 602.

When translocation occurs, an impedance value for an affected electrode (i.e., an electrode located on a portion of an electrode lead that has translocated) may change (e.g., increase) relative to the trend of other electrodes on the electrode lead. System 600 may accordingly be configured to monitor, during a lead insertion procedure in which an electrode lead having a plurality of electrodes is inserted into a cochlea of a recipient of a cochlear implant, impedance values for a subset of electrodes included in the plurality of electrodes. If system 600 detects, during the lead insertion procedure and based on the monitoring, an anomaly in the impedance values, system 600 may generate translocation log data indicating that a translocation event in which the electrode lead translocates from a first scala of the cochlea to a second scala of the cochlea is about to occur or has occurred during the lead insertion procedure.

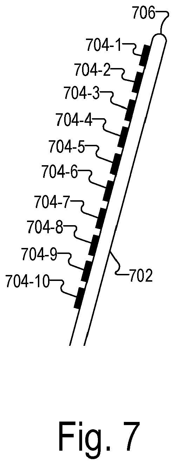

Various examples of electrode impedance value monitoring and generating translocation log data are described herein. In so doing, reference is made to FIG. 7, which shows a distal portion of an electrode lead 702 with a plurality of electrodes 704 (e.g., electrodes 704-1 through 704-10) disposed thereon. Electrode 704-1 is a distal-most electrode on lead 702 located closest to a distal end 706 of lead 702. The number of electrodes 704 shown as being disposed on lead 702 is exemplary only, and has been limited to simplify the following discussion. Any suitable number of electrodes 704 (e.g., sixteen) may be included on lead 702 without departing from the scope of the present disclosure. Electrode lead 702 and electrodes 704 may be similar to any of the electrode leads and electrodes described herein.

As mentioned, system 600 may be configured to monitor impedance values for a subset of electrodes on an electrode lead, as opposed to monitoring impedance values for all of the electrodes on the electrode lead. This may be faster and less resource intensive than conventional techniques that perform impedance measurements on all of the electrodes on the electrode lead. Any suitable number of electrodes may be included in the subset, as long as the number is less than the total number of electrodes on the electrode lead. For example, with respect to FIG. 7, the subset of electrodes may include up to nine of the ten total electrodes 704. As a specific example, the subset of electrodes may include only one, only two, only three, or only four electrodes.

In some examples, the subset of electrodes includes at least the distal most electrode 704-1. For example, the subset of electrodes may only include electrodes 704-1 through 704-4, which are closer to the distal end 106 than any other electrode on electrode lead 702.

Other groups of electrodes 704 may alternatively be included in the subset. For example, a group of electrodes in the middle of the array of electrodes 704 (e.g., electrodes 704-3 through 704-6) may be the only electrodes included in the subset of electrodes. Alternatively, non-adjacent electrodes may be the only electrodes included in the subset (e.g., electrodes 704-1, 704-3, 704-5, and 704-7).

In some examples, system 600 may dynamically update which electrodes 704 are selected for inclusion in the subset. For example, electrodes 704-1 through 704-4 may be initially selected by system 600 for inclusion in the subset. Subsequently, based on a change in one or more attributes of the lead insertion procedure, a different set of electrodes (e.g., electrodes 704-4 through 704-7) may be selected for inclusion in the subset.

System 600 may be configured to monitor impedance values for the subset of electrodes in any suitable manner. For example, system 600 may direct a cochlear implant to which electrode lead 702 is coupled to apply electrical stimulation (e.g., a stimulation pulse) by way of one or more electrodes 704 on electrode lead 702 and detect resultant voltages between the subset of electrodes 704 and a reference (e.g., a ground electrode on the electrode lead 702 and located outside the cochlea, a metal case of the cochlear implant, one of electrodes 704, etc.). The impedance values may be accordingly determined based on Ohm's law. Alternatively, it will be recognized that impedance values may be monitored simply by detecting voltages without actually calculating impedance values based on the detected voltages. Moreover, while reference is made in the examples herein to monitoring impedance values, any other physiologically generated signal may be monitored and used in a similar manner. In these cases, such physiologically generated signals may be measured by detecting voltages and/or other electrical characteristics. Hence, references made to monitoring impedances values herein may broadly refer to monitoring any physiologically generated signal using electrodes on an electrode lead. Such monitoring may be performed, for example, by measuring one or more voltages associated with the electrode (e.g., a voltage between an electrode and any of the references described herein, a voltage between two electrodes, etc.).

Various impedance measurement techniques that may be used by system 600 to detect impedance values for each electrode included in a subset of electrodes will now be described. It will be recognized that other impedance measurement techniques may additionally or alternatively be used by system 600 in accordance with the principles described herein.

In one example, the subset of electrodes for which system 600 monitors impedance values includes at least a first electrode (e.g., electrode 704-1), a second electrode (e.g., electrode 704-2), and a third electrode (e.g., electrode 704-2). In this example, system 600 may monitor the impedance values of the first, second, and third electrodes by sequentially and repeatedly measuring a first impedance value for the first electrode, a second impedance value for the second electrode, and a third impedance value for the third electrode in any of the ways described herein. Based on the second and third impedance values, system 600 may determine an expected impedance value for the first electrode. For example, system 600 may interpolate or otherwise process the second and third impedance values to establish the expected impedance value for the first electrode. It will be recognized that impedance values for any of the other electrodes in the subset may also be used to determine the expected value for the first electrode as may serve a particular implementation.

In this example, system 600 may be configured to detect an anomaly in the impedance values by comparing the first impedance value with the expected impedance value and determining, based on the comparison, that a difference between the first impedance value and the expected impedance value is greater than a threshold amount. For example, if the first impedance value is substantially higher than the expected impedance value, system 600 may output transaction log data indicating that the electrode lead (or at least the distal end of the electrode lead) is about to translocate or has translocated.

The threshold amount to which the difference between the first impedance value and the expected impedance value is compared may be set in any suitable manner. For example, system 600 may set the expected impedance value by establishing a baseline impedance value in any suitable manner, interpolating previous measured impedance values for the first electrode, determining a recipient-specific expected value based on one or more characteristics of the cochlea and/or electrode lead, etc.

As another example, system 600 may detect an impedance value for a particular electrode by directing a cochlear implant to apply electrical stimulation by way of the electrode and detect, in response to the application of the electrical stimulation by way of the electrode, a voltage between the electrode and a reference. The reference may be implemented in any of the ways described herein.

To illustrate, the subset of electrodes for which system 600 monitors impedance values may include at least a first electrode (e.g., electrode 704-1) and a second electrode (e.g., electrode 704-2). In this example, system 600 may monitor an impedance value of the first electrode by directing the cochlear implant to apply electrical stimulation by way of the first electrode and detecting, in response to the applying of the electrical stimulation by way of the first electrode, a voltage between the first electrode and a reference. Subsequently, system 600 may apply electrical stimulation by way of the second electrode and detect, in response to the applying of the electrical stimulation by way of the second electrode, a voltage between the second electrode and the reference. System 600 may similarly detect impedance values for other electrodes included in the subset.

Additionally or alternatively, system 600 may use a cross impedance measurement technique to determine the impedance values for the electrodes in the subset. Cross impedance measurement techniques are described more fully in PCT Publication No. WO2019/045747.

For example, system 600 may direct a cochlear implant to apply electrical stimulation on one electrode and record on (e.g., measure voltages at) the other electrodes in the subset, or apply electrical stimulation on all electrodes in the subset and record on another electrode (either in or out of the subset). System 600 may detect a decrease or an increase in cross-impedance (or a deviation from baseline established by more basal electrodes). For example, system 600 may stimulate on electrodes 704-1, 704-2, 704-3, and 704-4 and record on electrode 704-5. The recording may include measuring a voltage between electrode 704-5 and a reference (e.g., the electrode by which the electrical stimulation is applied or any of the other references described herein). Alternatively, system 600 may stimulate on 704-2, 704-3, 704-4, and 704-5 while recording on electrode 704-1. An advantage of this technique is that the recording electrode can remain constant, thereby speeding up the monitoring process.

In some examples, system 600 may measure an impedance value for a particular electrode (e.g., electrode 704-1) in the subset more frequently than impedance values for other electrodes (e.g., electrodes 704-2 through 704-4) included in the subset. Because switching between electrodes can be relatively slow, this may allow speed up of a most relevant acquisition sequence.

For example, in each measurement cycle, system 600 may determine which electrode to measure based on previous impedance value measurements. For example, electrode 704-1 can be recorded more frequently, while electrodes 704-2, 704-3, and 704-4 can be recorded less frequently and in an interleaved way. For example, the sequence can be as follows: 704-1, 704-1, 704-1, 704-2, 704-1, 704-1, 704-1, 704-3, 704-1, 704-1, 704-1, 704-4, etc.

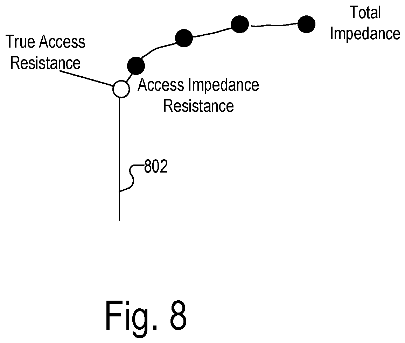

System 600 may measure an impedance of an electrode in any suitable manner. For example, system 600 may measure a simple resistance (single point), or measure multiple points using a "super-sampling" concept. To illustrate, FIG. 8 shows various types of resistances that may be measured by system 600. Line 802 represents a stimulation pulse applied by way of an electrode. System 600 may detect a true access impedance of the electrode (i.e., the "zero time" impedance that corresponds precisely to an occurrence of the pulse by measuring a few pulses with sampling offset between the pulses and then projecting down to the "zero time"). Alternatively, system 600 may measure the total impedance and/or determine an access resistance estimate in any suitable manner.

In some examples, system 600 may measure an impedance value for only a single electrode (e.g., 704-1). System 600 may compare the impedance of this electrode to past impedance values for the electrode. If the impedance value changes by more than a threshold amount, system 600 may provide a notification of possible translocation. Additionally or alternatively, when system 600 detects a change in impedance on the single electrode above a threshold, system 600 may dynamically add one or more additional electrodes to the subset of electrodes for use in the monitoring. For example, system 600 may begin detecting impedances of other electrodes in the subset (e.g., electrodes 704-2, 704-3, and 704-4) to establish baseline and make a more accurate measurement. In this manner, time and resources may be conserved.

Various ways in which system 600 may detect an anomaly in the impedance values of the electrodes included in the subset will now be described.

In one example, system 600 may detect an anomaly in the impedance values by comparing the impedance values to one or more baseline impedance values for the subset of electrodes and determining, based on the comparison, that one or more of the impedance values differs by more than a threshold amount from one or more of the one or more baseline impedance values.

The one or more baseline impedance values to which the measured impedance values are compared may be determined in any suitable manner. For example, system 600 may determine the one or more baseline impedance values based on one or more previous impedance value measurements. To illustrate, system 600 may measure impedance values of the electrodes included in the subset as the electrodes are first being inserted into the cochlea. These initial impedance value measurements may be used to establish the one or more baseline impedance values against which subsequent impedance value measurements are compared.

Additionally or alternatively, system 600 may determine the one or more baseline impedance values based on one or more expected impedance value measurements. To illustrate, system 600 may access historical impedance value measurement data acquired during previously performed lead insertion procedures. Based on this historical impedance value measurement data, system 600 may determine one or more impedance values that are to be expected for one or more electrodes at different insertion depths. These expected impedance values may accordingly be used as baseline impedance values against which impedance value measurements acquired during the present lead insertion procedure are compared.

Additionally or alternatively, system 600 may determine the one or more baseline impedance values based on a preoperative image of the cochlea of the recipient. The preoperative image may be acquired using CT scans, a digital volume tomography (DVT) system, magnetic resonance imaging (MRI), ultrasound imaging, and/or any other suitable medical imaging technique. Based on this preoperative image, system 600 may identify one or more characteristics (e.g., size characteristics, shape characteristics, etc.) of the cochlea that may affect electrode impedance values during the lead insertion procedure. Based on the identified one or more characteristics, system 600 may determine baseline impedance values against which impedance value measurements acquired during the present lead insertion procedure are compared.

Additionally or alternatively, system 600 may determine the one or more baseline impedance values based on one or more characteristics of the electrode lead and/or recipient. For example, the baseline impedance values may be determined based on a particular make and model of electrode lead being used during the lead insertion procedure. As another example, the baseline impedance values may be determined based on an age of the recipient, a gender of the recipient, a size of the recipient, a preoperative hearing assessment (e.g., an audiogram) of the recipient, a preoperative image (e.g., a CT and/or MRI scan of the recipient's cochlea, temporal bones, etc.), and/or any other characteristic of the recipient as may serve a particular implementation.

As mentioned, system 600 may detect an anomaly in the impedance values of one or more measured impedance values differs by more than a threshold amount from one or more baseline impedance values. The threshold amount may be set in any suitable manner. For example, the threshold amount may be set based on one or more characteristics of the electrode lead and/or recipient.

To illustrate, system 600 may use fixed thresholds for impedance increase, or thresholds that are specific to a particular electrode design, or a particular point in the insertion process. In some examples, the thresholds may vary depending on the particular type of electrode lead that is being used. For example, different thresholds may be used for pre-curved electrode leads than for naturally straight electrode leads. In some examples, evoked response-based measurements, described herein, are used for one type of electrode lead (e.g., straight electrode leads) while impedance-based measurements are used for another type of electrode lead (e.g., pre-curved electrode leads).

In some examples, system 600 may maintain data representative of multiple threshold amounts. For example, system 600 may use a first relatively low threshold amount to determine that a translocation event is about to occur. System 600 may use a second relatively high threshold amount to determine that a translocation event has occurred. To illustrate, a difference between a measured impedance value and a baseline impedance value may increase as the electrode lead starts on a translocation trajectory and then actually translocates. Hence, system 600 may provide a warning that a translocation event is about to occur if the difference between the measured impedance value and the baseline impedance value is above the first relatively low threshold amount. System 600 may then provide a warning that a translocation event has occurred if the difference between the measured impedance value and the baseline impedance value goes above the second relatively high threshold amount.

System 600 may be configured to perform any suitable operation based on translocation log data generated in response to detecting an anomaly in the impedance values.

For example, system 600 may present, based on the translocation log data, a notification that the translocation event is about to occur or has occurred during the lead insertion procedure. System 600 may present the notification in any suitable manner. For example, system 600 may display the notification by way of a display device (e.g., within a graphical user interface displayed by the display device). To illustrate, system 600 may display the notification by way of a display device within a microscope used by a surgeon to perform the lead insertion procedure. In some examples, the display device may be included in an augmented reality system that produces a predicted image of the electrode lead's current status based on the translocation log data. Additionally or alternatively, system 600 may present an audible sound representative of the notification.

Additionally or alternatively, system 600 may be configured to provide, based on the translocation log data, one or more instructions regarding how to correct and/or prevent the translocation event. For example, system 600 may provide (e.g., display on a display device) one or more steps that may be performed to at least partially retract the electrode lead from the cochlea and then correctly reinsert the electrode lead into the cochlea.

Additionally or alternatively, system 600 may be configured to stop (e.g., automatically) the lead insertion procedure based on the translocation log data. This may be performed in any suitable manner. For example, if a computer-assisted lead insertion system (e.g., a robotic lead insertion system) is being used to insert the electrode lead, system 600 may direct the computer-assisted lead insertion system to stop the lead insertion procedure (e.g., by transmitting one or more commands to the computer-assisted lead insertion system). System 600 may be further configured to transmit one or more additional commands to the computer-assisted lead insertion system to cause the electrode lead to be retracted until the translocation log data indicates that the translocation event is no longer occurring or that the translocation event is no longer about to occur.

In some examples, system 600 may use a machine learning model to perform any of the operations described herein. For example, system 600 may use a machine learning model to assist in generating the translocation log data.

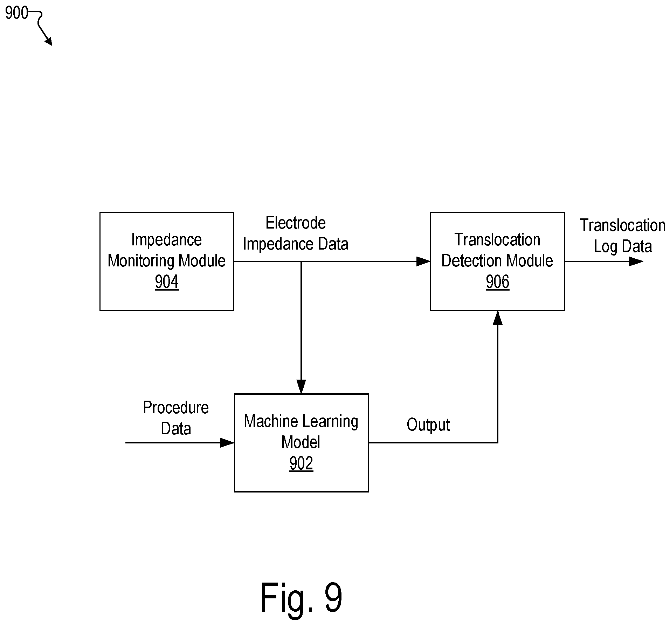

To illustrate, FIG. 9 shows an implementation 900 of system 600 in which system 600 uses a machine learning model 902 to assist in generating translocation log data. As shown, implementation 900 further includes an impedance monitoring module 904 and a translocation detection module 906. Modules 904 and 906 may be implemented by any suitable combination of hardware and/or software.

Impedance monitoring module 904 may be configured to perform any of the electrode impedance monitoring operations described herein. For example, impedance monitoring module 904 may be configured to monitor, during a lead insertion procedure in which an electrode lead having a plurality of electrodes is inserted into a cochlea of a recipient of a cochlear implant, impedance values for a subset of electrodes included in the plurality of electrodes. As shown, impedance monitoring module 904 may be configured to output electrode impedance data, which is representative of the impedance values of the subset of electrodes.

Translocation detection module 906 may be configured to perform any of the anomaly detection operations and translocation log data generation operations described herein. For example, as shown, translocation detection module 906 may generate translocation log data based on the electrode impedance data output by impedance monitoring module 904. In the particular example of FIG. 9, translocation detection module 906 may further base the generation of the translocation log data on an output of machine learning model 902.

Machine learning model 902 may be configured to perform any suitable machine learning heuristic (also referred to as artificial intelligence heuristic) with respect to various types of input data, which are described herein. Machine learning model 902 may be supervised and/or unsupervised as may serve a particular implementation and may be configured to implement one or more decision tree learning algorithms, association rule learning algorithms, artificial neural network learning algorithms, deep learning algorithms, bitmap algorithms, and/or any other suitable data analysis technique as may serve a particular implementation.

In some examples, machine learning model 902 may be implemented by one or more neural networks, such as one or more deep convolutional neural networks (CNN) using internal memories of its respective kernels (filters), recurrent neural networks (RNN), and/or long/short term memory neural networks (LSTM). Machine learning model 902 may be multi-layer. For example, machine learning model 902 may be implemented by a neural network that includes an input layer, one or more hidden layers, and an output layer.

System 600 may access machine learning model 902 in any suitable manner. For example, system 600 may store data representative of machine learning model 902 in memory 602. Additionally or alternatively, data representative of machine learning model 902 may be maintained by a system (e.g., one or more servers or other computing devices) remote from system 600. In these examples, system 600 may access machine learning model 902 by communicating with the remote system by way of a network.

As shown, system 600 may provide procedure data as an input to machine learning model 902. The procedure data input into machine learning model 902 may be representative of one or more contextual attributes of the lead insertion procedure for the recipient. Each of these contextual attributes may affect an outcome of the lead insertion procedure (e.g., by affecting a probability that a translocation event will occur during the lead insertion procedure). Various examples of contextual attributes that may be represented by procedure data will now be provided. It will be recognized that these examples are merely illustrative of the many different types of contextual attributes that may be represented by the procedure data as may serve a particular implementation.

In some examples, the procedure data may be representative of one or more characteristics (e.g., a make, model, type, size, flexibility rating, etc.) of the electrode lead being inserted into the cochlea and/or a tool being used to insert the electrode lead into the cochlea.

The procedure data may additionally or alternatively be representative of one or more characteristics of an opening in the recipient through which the electrode lead is to be inserted. For example, the procedure data may be representative of a location and/or a size of the opening.

The procedure data may additionally or alternatively be representative of an identity of a user performing or otherwise associated with the lead insertion procedure. For example, the procedure data may be representative of a user ID associated with a surgeon who performs the lead insertion procedure. This user ID may be used to access historical data associated with the user to determine one or more surgical tendencies of the user (e.g., electrode lead preferences, tool preferences, recipient positioning preferences, etc.). These tendencies may affect an outcome of the lead insertion procedure.

The procedure data may additionally or alternatively be representative of recipient-specific information. For example, the procedure data may be representative of a preoperative assessment (e.g., an audiogram) of a hearing profile of the recipient, an age of the recipient, a size of the recipient's cochlea, etc.

The procedure data may additionally or alternatively be representative of an insertion depth for the electrode lead, an insertion speed at which the electrode lead is inserted into the cochlea, and/or or an insertion angle at which the electrode lead is inserted into the cochlea. Such data may be determined preoperatively (e.g., based on historical data associated with a particular user). Additionally or alternatively, such data may be determined in real time during the lead insertion procedure in any suitable manner.

The procedure data may additionally or alternatively be representative of one or more preoperative images of the recipient's cochlea and/or a geometric model of the recipient's cochlea. The geometric model may be generated in any suitable manner (e.g., based on one or more preoperative images of the recipient's cochlea).

The procedure data may additionally or alternatively be representative of one or more intraoperative measurements performed with respect to the recipient during the lead insertion procedure. For example, the procedure data may be representative of a measurement of an evoked response elicited by stimulation (e.g., acoustic stimulation) of the recipient. Exemplary evoked responses include, but are not limited to, an electrocochleographic (ECochG) potential (e.g., a cochlear microphonic potential, a compound action potential such as an auditory nerve response, a summating potential, etc.), a brainstem response, a stapedius reflex, and/or any other type of neural or physiological response that may occur within a recipient in response to application of acoustic stimulation to the recipient. Evoked responses may originate from neural tissues, hair cell to neural synapses, inner or outer hair cells, and/or other sources.

The intraoperative measurement may additionally or alternatively include a measurement acquired by a sensor on the electrode lead. This sensor may include a force sensor, a pressure sensor, and/or any other type of sensor as may serve a particular implementation. For example, a force sensor and/or a pressure sensor may be configured to sense when the electrode lead is pressing against a wall of the cochlea.

The intraoperative measurement may additionally or alternatively include an ultrasound measurement, an optical sensor measurement, an electrical field sensor measurement, an electrode impedance measurement, and/or any other type of intraoperative measurement that may be performed by any suitable sensor and/or device.

The intraoperative measurement may additionally or alternatively include a scan of the cochlea that may be performed in any suitable manner. From this scan, rotational insertion of the electrode lead may be estimated through calculation of how far the linear extent of the electrode lead had made it around the curved wall of the cochlea.

Machine learning model 902 may be configured to process the procedure data in any suitable manner. Based on this processing, machine learning model 902 may provide an output that takes into account the procedure data. The output of machine learning model 902 may be in any suitable form and/or format and may be used by translocation detection module 906 to generate the translocation log data.

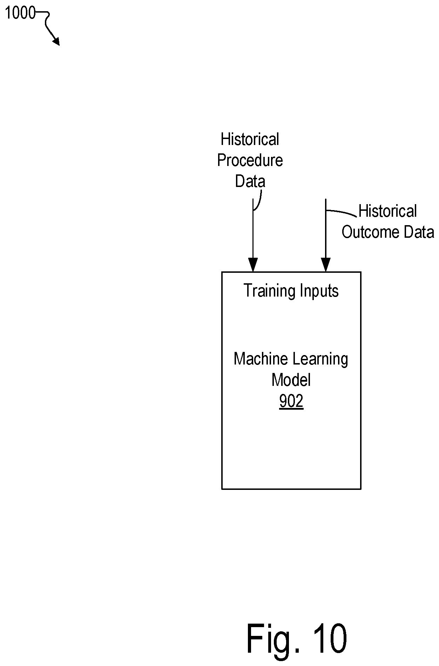

Machine learning model 902 may be trained by system 600 or any other system in any suitable manner. For example, FIG. 10 shows an exemplary configuration 1000 in which system 600 (or a different system remote from system 600) is configured to provide various types of data as training inputs to machine learning model 902. As shown, system 600 may provide historical procedure data and historical outcome data as training inputs to machine learning model 902.

Each of the historical data training inputs shown in FIG. 10 may correspond to a plurality of cochlear implant recipients and lead insertion procedures. For example, each of the historical data training inputs may include data collected over a period of time (e.g., years) of the insertion procedures for various cochlear implant recipients at one or more clinics and as performed by one or more users (e.g., surgeons). For example, the historical procedure data may be representative of contextual attributes of a plurality of lead insertion procedures in which electrode leads are inserted to the cochleas of the cochlear implant recipients and the historical outcome data may be representative of subjective and/or objective outcomes of the lead insertion procedures (e.g., lead insertion procedures in which translocation events did not occur and lead insertion procedures in which translocation events did occur). Based on this training data, machine learning model 902 may learn how various combinations of factors related to a lead insertion procedure may combine to determine whether a translocation event is about to occur or whether a translocation event has occurred.

In some examples, an indicator may be provided configured to indicate when insertion is started (e.g., impedance of electrode 704-1 becomes low), or when an electrode goes off a stylet or tube. During this starting time, a baseline can be established to which impedance measurements may be compared.

In some examples, the electrode lead, an insertion tool used to insert the electrode lead, and/or any other component may include a mechanism whereby moving off of stylet or tube can be detected, and impedance recording can be initiated.

In some examples, system 600 may dynamically switch between translocation detection techniques based on one or more factors. For example, in some scenarios, it may be that changing electrode contact on which impedance measurement is made takes some time. To avoid this issue, system 600 may record on a single electrode (e.g., electrode 704-1) only. If impedance on this electrode increases beyond a threshold value relative to a previous time period (e.g., last 2 seconds), system 600 may provide a stop signal to stop the lead insertion procedure. System 600 may then make full impedance measurement using multiple electrodes to confirm that this signal is indicative of translocation. If the full measurement determines that initial signal was a false alarm, system 600 may indicate to the surgeon to continue.

To further strengthen detection of translocation events, in cases when low frequency hearing is present in the recipient, system 600 may be configured to elicit and record one or more evoked responses (e.g., using configuration 400). This is described in more detail in PCT Publication No. WO2019/045680. If system 600 detects a change (e.g., a drop beyond a threshold) in the evoked response signal, system 600 may commence with any one of the impedance-based detection techniques described herein to further confirm true translocation so that false positives can be avoided. Similarly, impedance-based monitoring can be utilized first, with evoked response-based measurement being used by system 600 for confirmation. For example, system 600 may record an evoked response from the most apical electrode (e.g., electrode 704-1), and subsequently from a more basal electrode (e.g., electrode 704-3).

In some instances, an evoked response signal is weak at the base of the cochlea. Therefore, in some examples, system 600 may estimate depth of insertion and use impedance-based measurement at the beginning of insertion, and then transition to evoked response-based measurement for electrodes that are inserted at least a threshold distance into the cochlea. In some examples, system 600 may determine a speed at which electrodes are being inserted into the cochlea based on detected changes in impedance. This may allow system 600 ascertain where the electrode lead is in the cochlea.

In some examples, system 600 may measure several portions of an electric field imaging (EFI) matrix and combine the measured portions in a manner that detects when translocation occurs. This may be performed in any suitable manner.

Once actual translocation is detected by system 600, system 600 may automatically store the information for documentation and training purposes and the information can be stored in the fitting system for aid in the programming. This feature can be optionally turned off.

Following a lead insertion procedure, system 600 may run a full electrode impedance analysis for additional electrodes not included in the subset. This may confirm an occurrence of a translocation event and/or identify one or more other anomalies associated with the lead insertion procedure. This may be triggered in any suitable manner (e.g., by software or automatically).

Any of the measurement techniques and/or features described herein may be optionally enabled or disabled depending on a particular surgeon's insertion technique, time available, residual hearing in the ear, perceived anatomical risk prior to surgery, and/or any other factor. In some examples, system 600 may assess one or more of these factors and generate automatic recommendation based on the assessment. A user may accept or override the recommendation.

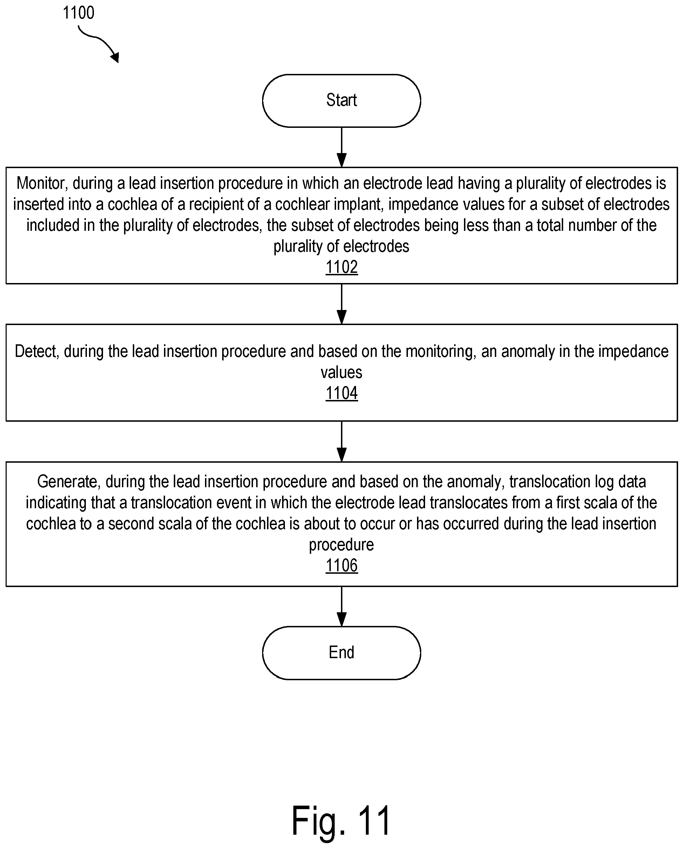

FIG. 11 illustrates an exemplary method 1100 that may be performed by an insertion management system (e.g., system 600 or any implementation thereof, such as at least one computing device). While FIG. 11 illustrates exemplary operations according to one embodiment, other embodiments may omit, add to, reorder, and/or modify any of the operations shown in FIG. 11. Each of the operations shown in FIG. 11 may be performed in any of the ways described herein.

At operation 1102, an insertion management system monitors, during a lead insertion procedure in which an electrode lead having a plurality of electrodes is inserted into a cochlea of a recipient of a cochlear implant, impedance values for a subset of electrodes included in the plurality of electrodes, the subset of electrodes being less than a total number of the plurality of electrodes.

At operation 1104, the insertion management system detects, during the lead insertion procedure and based on the monitoring, an anomaly in the impedance values.

At operation 1106, the insertion management system generates, during the lead insertion procedure and based on the anomaly, translocation log data indicating that a translocation event in which the electrode lead translocates from a first scala of the cochlea to a second scala of the cochlea is about to occur or has occurred during the lead insertion procedure.

In some examples, a non-transitory computer-readable medium storing computer-readable instructions may be provided in accordance with the principles described herein. The instructions, when executed by a processor of a computing device, may direct the processor and/or computing device to perform one or more operations, including one or more of the operations described herein. Such instructions may be stored and/or transmitted using any of a variety of known computer-readable media.

A non-transitory computer-readable medium as referred to herein may include any non-transitory storage medium that participates in providing data (e.g., instructions) that may be read and/or executed by a computing device (e.g., by a processor of a computing device). For example, a non-transitory computer-readable medium may include, but is not limited to, any combination of non-volatile storage media and/or volatile storage media. Exemplary non-volatile storage media include, but are not limited to, read-only memory, flash memory, a solid-state drive, a magnetic storage device (e.g. a hard disk, a floppy disk, magnetic tape, etc.), ferroelectric random-access memory ("RAM"), and an optical disc (e.g., a compact disc, a digital video disc, a Blu-ray disc, etc.). Exemplary volatile storage media include, but are not limited to RAM (e.g., dynamic RAM).

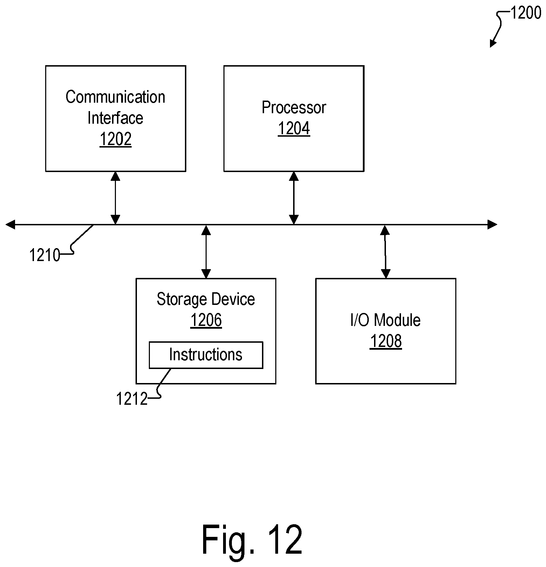

FIG. 12 illustrates an exemplary computing device 1200 that may be specifically configured to perform one or more of the processes described herein. To that end, any of the systems, processing units, and/or devices described herein may be implemented by computing device 1200.

As shown in FIG. 12, computing device 1200 may include a communication interface 1202, a processor 1204, a storage device 1206, and an input/output ("I/O") module 1208 communicatively connected one to another via a communication infrastructure 1210. While an exemplary computing device 1200 is shown in FIG. 12, the components illustrated in FIG. 12 are not intended to be limiting. Additional or alternative components may be used in other embodiments. Components of computing device 1200 shown in FIG. 12 will now be described in additional detail.

Communication interface 1202 may be configured to communicate with one or more computing devices. Examples of communication interface 1202 include, without limitation, a wired network interface (such as a network interface card), a wireless network interface (such as a wireless network interface card), a modem, an audio/video connection, and any other suitable interface.

Processor 1204 generally represents any type or form of processing unit capable of processing data and/or interpreting, executing, and/or directing execution of one or more of the instructions, processes, and/or operations described herein. Processor 1204 may perform operations by executing computer-executable instructions 1212 (e.g., an application, software, code, and/or other executable data instance) stored in storage device 1206.

Storage device 1206 may include one or more data storage media, devices, or configurations and may employ any type, form, and combination of data storage media and/or device. For example, storage device 1206 may include, but is not limited to, any combination of the non-volatile media and/or volatile media described herein. Electronic data, including data described herein, may be temporarily and/or permanently stored in storage device 1206. For example, data representative of computer-executable instructions 1212 configured to direct processor 1204 to perform any of the operations described herein may be stored within storage device 1206. In some examples, data may be arranged in one or more databases residing within storage device 1206.

I/O module 1208 may include one or more I/O modules configured to receive user input and provide user output. I/O module 1208 may include any hardware, firmware, software, or combination thereof supportive of input and output capabilities. For example, I/O module 1208 may include hardware and/or software for capturing user input, including, but not limited to, a keyboard or keypad, a touchscreen component (e.g., touchscreen display), a receiver (e.g., an RF or infrared receiver), motion sensors, and/or one or more input buttons.

I/O module 1208 may include one or more devices for presenting output to a user, including, but not limited to, a graphics engine, a display (e.g., a display screen), one or more output drivers (e.g., display drivers), one or more audio speakers, and one or more audio drivers. In certain embodiments, I/O module 1208 is configured to provide graphical data to a display for presentation to a user. The graphical data may be representative of one or more graphical user interfaces and/or any other graphical content as may serve a particular implementation.

In the preceding description, various exemplary embodiments have been described with reference to the accompanying drawings. It will, however, be evident that various modifications and changes may be made thereto, and additional embodiments may be implemented. For example, certain features of one embodiment described herein may be combined with or substituted for features of another embodiment described herein. The description and drawings are accordingly to be regarded in an illustrative rather than a restrictive sense.

## Claims

1. A system comprising:
a memory (112, 602) storing instructions (606);
a processor (114, 202, 604) communicatively coupled to the memory and configured to execute the instructions to:
monitor, during a lead insertion procedure in which an electrode lead (104, 504, 702) having a plurality of electrodes (106, 512, 704) is inserted into a cochlea (506) of a recipient of a cochlear implant (102), impedance values for a subset of electrodes included in the plurality of electrodes, the subset of electrodes being less than a total number of the plurality of electrodes;
detect, during the lead insertion procedure and based on the monitoring, an anomaly in the impedance values; **characterised in that** said processor is further configured to execute the instructions to
generate, during the lead insertion procedure and based on the anomaly, translocation log data indicating that a translocation event in which the electrode lead translocates from a first scala (516) of the cochlea to a second scala (520) of the cochlea is about to occur or has occurred during the lead insertion procedure.

2. The system of claim 1, wherein the processor (114, 202, 604) is further configured to execute the instructions (606) to present, based on the translocation log data, a notification that the translocation event is about to occur or has occurred during the lead insertion procedure.

3. The system of claim 1, wherein the monitoring of the impedance values comprises:
directing the cochlear implant (102) to apply electrical stimulation by way of one or more electrodes (106, 512, 704) on the electrode lead (104, 504, 702); and
detecting voltages between the subset of electrodes and a reference.

4. The system of claim 1, wherein:
the subset of electrodes (106, 512, 704) comprises a first electrode (704-1), a second electrode (704-2), and a third electrode (704-3); and
the monitoring of the impedance values comprises:
sequentially and repeatedly measuring a first impedance value for the first electrode, a second impedance value for the second electrode, and a third impedance value for the third electrode, and
determining, based on the second and third impedance values, an expected impedance value for the first electrode.

5. The system of claim 4, wherein the detecting of the anomaly in the impedance values comprises:
comparing the first impedance value with the expected impedance value; and
determining, based on the comparing, that a difference between the first impedance value and the expected impedance value is greater than a threshold amount.

6. The system of claim 1, wherein:
the subset of electrodes (106, 512, 704) comprises a first electrode (704-1) and a second electrode (704-2); and
the monitoring of the impedance values comprises:
directing the cochlear implant (102) to apply electrical stimulation by way of the first electrode;
detecting, in response to the applying of the electrical stimulation by way of the first electrode, a voltage between the first electrode and a reference;
directing, subsequent to the applying of the electrical stimulation by way of the first electrode, the cochlear implant to apply the electrical stimulation by way of the second electrode; and
detecting, in response to the applying of the electrical stimulation by way of the second electrode, a voltage between the second electrode and the reference.

7. The system of claim 1, wherein:
the subset of electrodes (106, 512, 704) comprises a first electrode (704-1) and a second electrode (704-2); and
the monitoring of the impedance values comprises:
directing the cochlear implant (102) to apply electrical stimulation by way of the first electrode, and
detecting, in response to the applying of the electrical stimulation by way of the first electrode, a voltage between the second electrode and a reference.

8. The system of claim 1, wherein the monitoring of the impedance values comprises measuring an impedance value for a first electrode (704-1) included in the subset of electrodes more frequently than impedance values for other electrodes included in the subset of electrodes (106, 512, 704).

9. The system of claim 8, wherein the first electrode (704-1) is closer to a distal end of the electrode lead (104, 504, 702) than the other electrodes included in the subset of electrodes (106, 512, 704).

10. The system of claim 8, wherein the processor (114, 202, 604) is further configured to determine which electrode in the subset of electrodes (106, 512, 704) is the first electrode (704-1) based on one or more previous impedance value measurements.

11. The system of claim 1, wherein the detecting of the anomaly in the impedance values comprises:
comparing the impedance values to one or more baseline impedance values for the subset of electrodes (106, 512, 704); and
determining, based on the comparing, that one or more of the impedance values differs by more than a threshold amount from one or more of the one or more baseline impedance values.

12. The system of claim 11, wherein the processor (114, 202, 604) is further configured to execute the instructions (606) to determine the one or more baseline impedance values based on one or more previous impedance value measurements.

13. The system of claim 11, wherein the processor (114, 202, 604) is further configured to execute the instructions (606) to determine the one or more baseline impedance values based on one or more expected impedance value measurements.

14. The system of claim 11, wherein the processor (114, 202, 604) is further configured to execute the instructions (606) to determine the one or more baseline impedance values based on a preoperative image of the cochlea (506).

15. The system of claim 11, wherein the processor (114, 202, 604) is further configured to execute the instructions (606) to set the threshold amount based on at least one of a characteristic of the electrode lead (104, 504, 702) and a characteristic of the recipient.

## Patentansprüche

1. System mit:
einem Speicher (112, 602), welcher Befehle (606) speichert;
einem Prozessor (114, 202, 604), der kommunikativ mit dem Speicher gekoppelt ist, und ausgebildet ist, um die Befehle auszuführen, um:
während einer Zuleitungseinführprozedur, in welcher eine Elektrodenzuleitung (104, 504, 702) mit einer Mehrzahl von Elektroden (106, 512, 704) in eine Cochlea (506) eines Rezipienten eines Cochlea-Implantats (102) eingeführt wird, Impedanzwerte für eine Untermenge von in der Mehrzahl von Elektroden beinhalteten Elektroden zu überwachen, wobei die Untermenge von Elektroden kleiner als die Gesamtzahl der Mehrzahl von Elektroden ist;
während der Zuleitungseinführprozedur und basierend auf der Überwachung eine Anomalie in den Impedanzwerten zu erfassen; **dadurch gekennzeichnet, dass** der Prozessor ferner ausgebildet ist, um die Befehle auszuführen, um
während der Zuleitungseinführprozedur und basierend auf der Anomalie, Verlagerungs-Log-Daten zu erzeugen, die anzeigen, dass ein Verlagerungsereignis, in welchem die Elektrodenzuleitung von einer ersten Skala (516) der Cochlea zu einer zweiten Skala (520) der Cochlea verlagert wird, erfolgen wird oder während der Zuleitungseinführprozedur erfolgt ist.

2. System gemäß Anspruch 1, wobei der Prozessor (114, 202, 604) ferner ausgebildet ist, um die Befehle (606) auszuführen, um basierend auf dem Verlagerungs-Log-Daten eine Benachrichtigung zu präsentieren, dass das Verlagerungsereignis auftreten wird oder während der Zuleitungseinführprozedur aufgetreten ist.

3. System gemäß Anspruch 1, wobei beim Überwachen der Impedanzwerte das Cochlea-Implantat (102) veranlasst wird, elektrische Stimulation mittels mindestens einer Elektrode (106, 512, 704) auf der Elektrodenzuleitung (104, 504, 702) anzuwenden und Spannungen zwischen der Untermenge der Elektroden und einer Referenz erfasst werden.

4. System gemäß Anspruch 1, wobei:
die Untermenge von Elektroden (106, 512, 704) eine erste Elektrode (704-1), eine zweite Elektrode (704-2) und eine dritte Elektrode (704-3) aufweist; und
das Überwachen der Impedanzwerte beinhaltet, dass:
ein erster Impedanzwert für die erste Elektrode, ein zweiter Impedanzwert für die zweite Elektrode sowie ein dritter Impedanzwert für die dritte Elektrode sequentiell und wiederholt gemessen werden, und
basierend auf dem zweiten und dritten Impedanzwert ein erwarteter Impedanzwert für die erste Elektrode bestimmt wird.

5. System gemäß Anspruch 4, wobei beim Erfassen der Anomalie in den Impedanzwerten:
der erste Impedanzwert mit dem erwarteten Impedanzwert verglichen werden; und
basierend auf dem Vergleich bestimmt wird, dass eine Differenz zwischen dem ersten Impedanzwert und dem erwarteten Impedanzwert größer als ein Schwellenbetrag ist.

6. System gemäß Anspruch 1, wobei:
die Untermenge von Elektroden (106, 512, 704) eine erste Elektrode (704-1) und eine zweite Elektrode (704-2) aufweist; und
beim Überwachen der Impedanzwerte:
das Cochlea-Implantat (102) veranlasst wird, elektrische Stimulation mittels der ersten Elektrode anzuwenden;
als Reaktion auf das Anwenden der elektrischen Stimulation mittels der ersten Elektrode eine Spannung zwischen der ersten Elektrode und einer Referenz erfasst wird;
nachfolgend zu dem Anwenden der elektrischen Stimulation mittels der ersten Elektrode das Cochlea-Implantat veranlasst wird, die elektrische Stimulation mittels der zweiten Elektrode anzuwenden; und
als Reaktion auf das Anwenden der elektrischen Stimulation mittels der zweiten Elektrode eine Spannung zwischen der zweiten Elektrode und der Referenz erfasst wird.

7. System gemäß Anspruch 1, wobei:
die Untermengen von Elektroden (106, 512, 704) eine erste Elektrode (704-1) und eine zweite Elektrode (704-2) aufweist; und
beim Überwachen der Impedanzwerte:
das Cochlea-Implantat (102) veranlasst wird, elektrische Stimulation mittels der ersten Elektrode anzuwenden, und
als Reaktion auf das Anwenden der elektrischen Stimulation mittels der ersten Elektrode eine Spannung zwischen der zweiten Elektrode und einer Referenz erfasst wird.

8. System gemäß Anspruch 1, wobei beim Überwachen der Impedanzwerte ein Impedanzwert für eine erste Elektrode (704-1) in der Untermenge von Elektroden häufiger gemessen wird als Impedanzwerte für andere Elektroden in der Untermenge von Elektroden (106, 512, 704).

9. System gemäß Anspruch 1, wobei die erste Elektrode (704-1) näher an einem distalen Ende der Elektrodenzuleitung (104, 504, 702) liegt als die anderen Elektroden in der Untermenge von Elektroden (106, 512, 704).

10. System gemäß Anspruch 8, wobei der Prozessor (114, 202, 604) ferner ausgebildet ist, um basierend auf mindestens einer vorangegangenen Impedanzwertmessung zu bestimmen, welche Elektrode in der Untermenge von Elektroden (106, 512, 704) die erste Elektrode (704-1) bildet.

11. System gemäß Anspruch 1, wobei beim Erfassen der Anomalie in den Impedanzwerten:
die Impedanzwerte mit mindestens einem Basisimpendanzwert für die Untermenge von Elektroden (106, 512, 704) verglichen werden; und
basierend auf dem Vergleich festgestellt wird, dass sich mindestens einer der Impedanzwerte um mehr als einen Schwellenbetrag von dem Basisimpedanzwert oder mindestens einem der Basisimpedanzwerte unterscheidet.

12. System gemäß Anspruch 11, wobei der Prozessor (114, 202, 604) ferner ausgebildet ist, um die Befehle (606) zum Bestimmen des mindestens einen Basisimpedanzwertes basierend auf mindestens einer vorangegangenen Impedanzwertmessung auszuführen.

13. System gemäß Anspruch 11, wobei der Prozessor (114, 202, 604) ferner ausgebildet ist, um die Befehle zum Bestimmen des mindestens einen Basisimpedanzwertes basierend auf mindestens einer Messung für erwartete Impedanzwerte auszuführen.

14. System gemäß Anspruch 11, wobei der Prozessor (114, 202, 604) ferner ausgebildet ist, um die Befehle (606) zum Bestimmen des mindestens einen Basisimpedanzwertes basierend auf einem Voroperationsbild der Cochlea (506) auszuführen.

15. System gemäß Anspruch 11, wobei der Prozessor (114, 202, 604) ferner ausgebildet ist, um die Befehle zum Setzen des Schwellbetrags basierend auf mindestens einer Charakteristik der Elektrodenzuleitung (104, 504, 702) und einer Charakteristik des Rezipienten auszuführen.

## Revendications

1. Système comprenant :
une mémoire (112, 602) qui stocke des instructions (606) ;
un processeur (114, 202, 604) qui est couplé en communication à la mémoire et qui est configuré pour exécuter les instructions pour :
surveiller, pendant une procédure d'insertion de fil au cours de laquelle un fil d'électrodes (104, 504, 702) qui comporte une pluralité d'électrodes (106, 512, 704) est inséré à l'intérieur d'une cochlée (506) d'un bénéficiaire d'un implant cochléaire (102), des valeurs d'impédance pour un sous-ensemble d'électrodes qui sont incluses dans la pluralité d'électrodes, le sous-ensemble d'électrodes comprenant un nombre d'électrodes inférieur à un nombre total de la pluralité d'électrodes ; et
détecter, pendant la procédure d'insertion de fil et sur la base de la surveillance, une anomalie dans les valeurs d'impédance ;
**caractérisé en ce que** ledit processeur est en outre configuré pour exécuter les instructions pour générer, pendant la procédure d'insertion de fil et sur la base de l'anomalie, des données de journalisation de translocation qui indiquent qu'un événement de translocation au cours duquel le fil d'électrodes réalise une translocation depuis une première rampe (516) de la cochlée jusqu'à une seconde rampe (520) de la cochlée est sur le point de se produire ou s'est produit pendant la procédure d'insertion de fil.

2. Système selon la revendication 1, dans lequel le processeur (114, 202, 604) est en outre configuré pour exécuter les instructions (606) pour présenter, sur la base des données de journalisation de translocation, une notification du fait que l'événement de translocation est sur le point de se produire ou s'est produit pendant la procédure d'insertion de fil.

3. Système selon la revendication 1, dans lequel la surveillance des valeurs d'impédance comprend :
la commande à l'implant cochléaire (102) d'appliquer une stimulation électrique au moyen d'une ou de plusieurs électrodes (106, 512, 704) sur le fil d'électrodes (104, 504, 702) ; et
la détection de tensions entre le sous-ensemble d'électrodes et une référence.

4. Système selon la revendication 1, dans lequel :
le sous-ensemble d'électrodes (106, 512, 704) comprend une première électrode (704-1), une deuxième électrode (704-2) et une troisième électrode (704-3) ; et
la surveillance des valeurs d'impédance comprend :
la mesure de façon séquentielle et répétée d'une première valeur d'impédance pour la première électrode, d'une deuxième valeur d'impédance pour la deuxième électrode et d'une troisième valeur d'impédance pour la troisième électrode ; et
la détermination, sur la base des deuxième et troisième valeurs d'impédance, d'une valeur d'impédance attendue pour la première électrode.

5. Système selon la revendication 4, dans lequel la détection de l'anomalie dans les valeurs d'impédance comprend :
la comparaison de la première valeur d'impédance avec la valeur d'impédance attendue ; et
la détermination, sur la base de la comparaison, du fait qu'une différence entre la première valeur d'impédance et la valeur d'impédance attendue est supérieure à un niveau de seuil.

6. Système selon la revendication 1, dans lequel :
le sous-ensemble d'électrodes (106, 512, 704) comprend une première électrode (704-1) et une seconde électrode (704-2) ; et
la surveillance des valeurs d'impédance comprend :
la commande à l'implant cochléaire (102) d'appliquer une stimulation électrique au moyen de la première électrode ;
la détection, en réponse à l'application de la stimulation électrique au moyen de la première électrode, d'une tension entre la première électrode et une référence ;
la commande, subséquemment à l'application de la stimulation électrique au moyen de la première électrode, à l'implant cochléaire d'appliquer la stimulation électrique au moyen de la seconde électrode ; et
la détection, en réponse à l'application de la stimulation électrique au moyen de la seconde électrode, d'une tension entre la seconde électrode et la référence.

7. Système selon la revendication 1, dans lequel :
le sous-ensemble d'électrodes (106, 512, 704) comprend une première électrode (704-1) et une seconde électrode (704-2) ; et
la surveillance des valeurs d'impédance comprend :
la commande à l'implant cochléaire (102) d'appliquer une stimulation électrique au moyen de la première électrode ; et
la détection, en réponse à l'application de la stimulation électrique au moyen de la première électrode, d'une tension entre la seconde électrode et une référence.

8. Système selon la revendication 1, dans lequel la surveillance des valeurs d'impédance comprend la mesure d'une valeur d'impédance pour une première électrode (704-1) qui est incluse dans le sous-ensemble d'électrodes plus fréquemment que la mesure de valeurs d'impédance pour d'autres électrodes qui sont incluses dans le sous-ensemble d'électrodes (106, 512, 704).

9. Système selon la revendication 8, dans lequel la première électrode (704-1) est plus près d'une extrémité distale du fil d'électrodes (104, 504, 702) que les autres électrodes qui sont incluses dans le sous-ensemble d'électrodes (106, 512, 704).

10. Système selon la revendication 8, dans lequel le processeur (114, 202, 604) est en outre configuré pour déterminer quelle électrode dans le sous-ensemble d'électrodes (106, 512, 704) est la première électrode (704-1) sur la base d'une ou de plusieurs mesures de valeur d'impédance précédentes.

11. Système selon la revendication 1, dans lequel la détection de l'anomalie dans les valeurs d'impédance comprend :
la comparaison des valeurs d'impédance avec une ou plusieurs valeurs d'impédance de ligne de base pour le sous-ensemble d'électrodes (106, 512, 704) ; et
la détermination, sur la base de la comparaison, du fait qu'une ou plusieurs des valeurs d'impédance diffère(nt) de plus qu'un niveau de seuil d'une ou de plusieurs des une ou plusieurs valeurs d'impédance de ligne de base.

12. Système selon la revendication 11, dans lequel le processeur (114, 202, 604) est en outre configuré pour exécuter les instructions (606) pour déterminer les une ou plusieurs valeurs d'impédance de ligne de base sur la base d'une ou de plusieurs mesures de valeur d'impédance précédentes.

13. Système selon la revendication 11, dans lequel le processeur (114, 202, 604) est en outre configuré pour exécuter les instructions (606) pour déterminer les une ou plusieurs valeurs d'impédance de ligne de base sur la base d'une ou de plusieurs mesures de valeur d'impédance attendues.

14. Système selon la revendication 11, dans lequel le processeur (114, 202, 604) est en outre configuré pour exécuter les instructions (606) pour déterminer les une ou plusieurs valeurs d'impédance de ligne de base sur la base d'une image préopératoire de la cochlée (506).

15. Système selon la revendication 11, dans lequel le processeur (114, 202, 604) est en outre configuré pour exécuter les instructions (606) pour définir le niveau de seuil sur la base d'au moins une caractéristique parmi une caractéristique du fil d'électrodes (104, 504, 702) et une caractéristique du bénéficiaire.
